# EUROPEAN PATENT SPECIFICATION

(11) **EP 2 077 895 B1**
(45) Date of publication and mention of the grant of the patent: **02.09.2015**
(21) Application number: 07852602.7
(22) Date of filing: 09.10.2007
(51) Int. Cl.: A61N 1/36

(54) **IMPLANTABLE NEUROSTIMULATOR FOR MODULATING CARDIOVASCULAR FUNCTION**
IMPLANTIERBARER NEUROSTIMULATOR ZUR MODULATION DER HERZ-KREISLAUF-FUNKTION
NEUROSTIMULATEUR IMPLANTABLE DESTINÉ À MODULER LA FONCTION CARDIOVASCULAIRE

(30) Priority: 11.10.2006 US 548354
(43) Date of publication of application: 15.07.2009
(73) Proprietor: Cardiac Pacemakers, Inc., St. Paul MN 55112-5798 (US)
(72) Inventor: CAPARSO, Anthony, V., San Jose, CA 95134 (US); LIBBUS, Imad, St. Paul, MN 55105 (US); KRAMER, Andrew, P., Marine on St. Croix, MN 55047 (US)
(74) Representative: Golkowsky, Stefan
(86) International application number: PCT/US2007/021570
(87) International publication number: WO 2008/045434

(56) References cited:
- US-A- 5 456 708
- US-A1- 2003 040 774
- US-A1- 2004 220 621
- US-A1- 2005 038 491
- US-A1- 2006 161 208

## Description

This document relates generally to neurostimulation and particularly to a system and method for modulating cardiovascular function using neurostimulation delivered by an implantable neurostimulator.

### BACKGROUND

Neurostimulation has been applied or proposed to modulate various physiologic functions and treat various diseases. One example is the modulation of cardiovascular functions by stimulating sympathetic and parasympathetic nerves that innervate the heart. Activities in the vagus nerve, including artificially applied electrical stimuli, modulate the heart rate and contractility (strength of the myocardial contractions). Electrical stimulation applied to the vagus nerve is known to decrease the heart rate and the contractility, lengthening the diastolic phase of a cardiac cycle. This ability of the vagal nerve stimulation may be utilized, for example, to control myocardial remodeling. Electrical stimulation applied at acupuncture points is also known to have therapeutic effects in cardiovascular functions.

Neurostimulation provides therapeutic benefit when applied shortly after the occurrence of a cardiac disorder event such as acute myocardial infarction (MI). For example, after the acute MI, adverse ventricular remodeling starts and the heart is more susceptible to arrhythmias. Neurostimulation may be applied to control the post-MI ventricular remodeling and prevent the arrhythmias from occurring. For prompt deployment of a neurostimulation system following a cardiac disorder event such as acute MI, and for other reasons, there is a need for a neurostimulator that is implantable using a minimally invasive procedure. A device according to the preamble of claim 1 is known from US5456708.

### SUMMARY

The invention is defined in claim 1. A miniature implantable medical device includes an implantable capsule housing a circuit that delivers neurostimulation to modulate one or more cardiovascular functions. To limit displacement after implantation, a fixation device is coupled to the implantable capsule to fix the miniature implantable medical device to a position in the body of a patient.

In one embodiment, an implantable medical device includes electronic circuitry for delivering neurostimulation to modulate a cardiovascular function. The electronic circuitry includes a stimulation output circuit and a stimulation controller. The stimulation output circuit delivers the neurostimulation. The stimulation controller controls the delivery of the neurostimulation by executing a stimulation algorithm for modulating the cardiovascular function. The implantable medical device also includes an implantable capsule including a wall forming a chamber that houses the electronic circuitry. The implantable capsule has an approximately cylindrical elongate body. A fixation device is coupled to the implantable capsule to fix the position of the implantable capsule in a body.

In one embodiment, an implantable medical device includes electronic circuitry, an implantable capsule including a wall forming a chamber to house the electronic circuitry, and a transmural fixation device. The transmural fixation device transmurally fixes the implantable capsule to an internal structure of a body. The internal structure has a cavity and a wall. The wall has an exterior surface and an interior surface. The interior surface defines the cavity. The transmural fixation device includes a proximal end, a distal end, and an elongate transmural body between the proximal end and the distal end. The proximal end is coupled to the implantable capsule. The distal end includes an anchoring device, and is configured to pierce the wall from the exterior surface to enter the cavity such that the anchoring device is deployed in the cavity against the interior surface.

In one embodiment, a method for making an implantable medical device that modulates cardiovascular function is provided. Electronic circuitry capable of delivering neurostimulation is provided. The electronic circuitry is encapsulated in an approximately cylindrical implantable capsule. A fixation device is coupled to the implantable capsule. The fixation device is to fix the implantable capsule to a location in a body. The electronic circuitry is programmed for controlling the delivery of the neurostimulation by executing a stimulation algorithm adapted to modulate a cardiovascular function.

In one embodiment, a method for making an implantable medical device with a fixation device is provided. Electronic circuitry is provided and encapsulated in an implantable capsule. A transmural fixation device is provided to transmurally fix the implantable capsule to an internal structure of a body. The internal structure has a cavity and a wall. The wall has an exterior surface and an interior surface. The interior surface defines the cavity. The transmural fixation device includes a proximal end, a distal end, and an elongate transmural body between the proximal end and the distal end. The distal end includes an anchoring device and is configured to pierce the wall from the exterior surface to enter the cavity such that the anchoring device is deployed in the cavity against the interior surface. The proximal end is coupled to the implantable capsule.

This Summary is an overview of some of the teachings of the present application and not intended to be an exclusive or exhaustive treatment of the present subject matter. Further details about the present subject matter are found in the detailed description and appended claims. Other aspects of the invention will be apparent to persons skilled in the art upon reading and understanding the following detailed description and viewing the drawings that form a part thereof. The scope of the present invention is defined by the appended claims and their legal equivalents.

### BRIEF DESCRIPTION OF THE DRAWINGS

The drawings illustrate generally, by way of example, various embodiments discussed in the present document. The drawings are for illustrative purposes only and may not be to scale.
FIG. 1 is an illustration of an embodiment of a neurostimulation system and portions of an environment in which the neurostimulation system is used.
FIG. 2 is an illustration of another embodiment of the neurostimulation system and portions of an environment in which the neurostimulation system is used.
FIG. 3 is an illustration of an embodiment of a miniature implantable medical device of the neurostimulation system.
FIG. 4 is another illustration of the embodiment of the implantable medical device.
FIG. 5 is a block diagram illustrating an embodiment of a circuit of the implantable medical device.
FIG. 6 is an illustration of an embodiment of the implantable medical device with a suture loop.
FIGS. 7A-B are illustrations of an embodiment of the implantable medical device with a fixation cuff.
FIG. 8 is an illustration of another embodiment of the implantable medical device with a fixation cuff.
FIG. 9 is an illustration of another embodiment of the implantable medical device with a fixation cuff.
FIG. 10 is an illustration of another embodiment of the implantable medical device with a fixation cuff.
FIGS. 11A-B are illustrations of another embodiment of the implantable medical device with a fixation cuff and portions of a structure to which the implantable medical device is fixed.
FIG. 12 is an illustration of another embodiment of the implantable medical device with a fixation cuff.
FIG. 13A is an illustration of an embodiment of the implantable medical device with a transmural fixation device.
FIG. 13B is an illustration of another embodiment of the implantable medical device with the transmural fixation device of FIG. 13A.
FIGS. 14A is an illustration of another embodiment of the implantable medical device with a transmural fixation device.
FIGS. 14B is an illustration of another embodiment of the implantable medical device with the transmural fixation device of FIG. 14A.
FIGS. 15A-C are illustrations of an embodiment of a method for fixing the position of the implantable medical device using the transmural fixation device of FIGS. 14A-B.
FIG. 16 is a flow chart illustrating a method for making a miniature implantable medical device.

### DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the spirit and scope of the present invention. References to "an", "one", or "various" embodiments in this disclosure are not necessarily to the same embodiment, and such references contemplate more than one embodiment. The following detailed description provides examples, and the scope of the present invention is defined by the appended claims and their legal equivalents.

This document discusses a system that includes a miniature implantable medical device that modulates cardiovascular function by delivering neurostimulation. The miniature implantable medical device (also known as microstimulator) is a self-contained device that includes an implantable capsule housing a circuit that delivers neurostimulation and/or senses a physiologic signal through electrodes. To limit displacement after implantation, a fixation device coupled to the implantable capsule fixes the miniature implantable medical device to a position in the body of a patient.

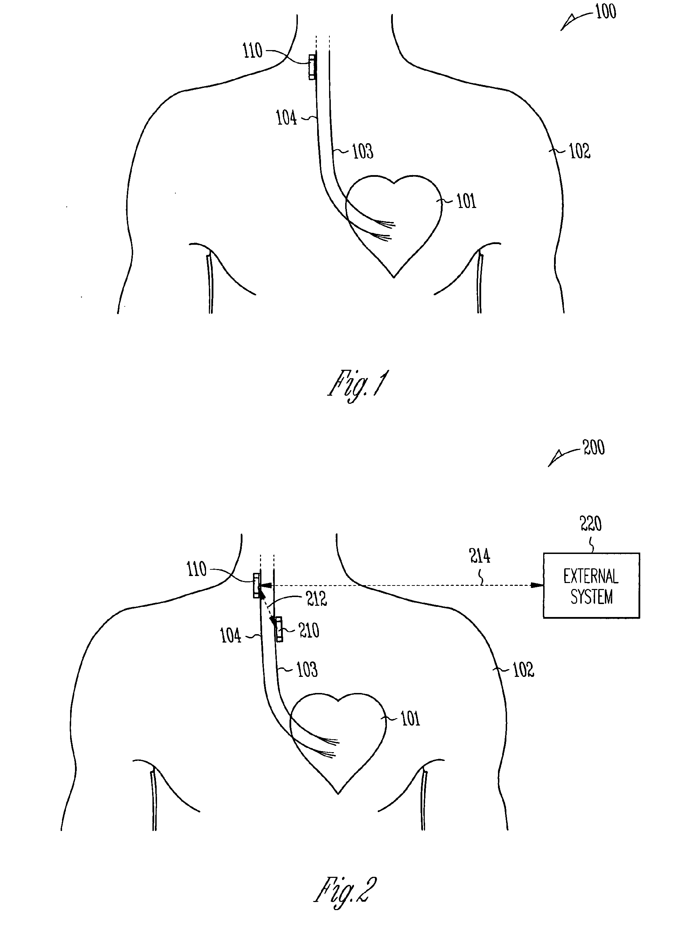

FIG. 1 is an illustration of an embodiment of a neurostimulation system 100 and portions of an environment in which system 100 is used. System 100 includes a miniature implantable medical device 110 that delivers neurostimulation to a body 102 having a heart 101 and nerves 103 and 104 that innervate heart 101. In one example for illustrative purposes only, nerve 103 represents a nerve of the sympathetic nervous system, and nerve 104 represents a nerve of the parasympathetic nervous system. In one embodiment, implantable medical device 110 delivers neurostimulation to any component of the nervous system whose activities affect one or more cardiovascular functions. Examples of such component of the nervous system include baroreceptors, aortic nerve, carotid nerve, vagus nerve, the spinal cord dorsal or ventral nerves, and the sympathetic ganglia and nerves.

Implantable medical device 110 delivers neurostimulation by executing a stimulation algorithm for modulating a cardiovascular function. In the illustrated embodiment, implantable medical device 110 is implanted in a vicinity of parasympathetic nerve 104. By controlling stimulation parameters, the neurostimulation is delivered to increase or decrease the parasympathetic tone. In one embodiment, implantable medical device 110 is also capable of sensing physiological signals, such as neural activities in nerve 104 or cardiac electric signals.

FIG. 2 is an illustration of an embodiment of a neurostimulation system 200 and portions of an environment in which system 200 is used. System 200 includes implantable medical device 110, another miniature implantable medical device 210, an external system 220, a telemetry link 212 providing for communication between implantable medical devices 110 and 210, and another telemetry link 214 providing for communication between implantable medical devices 110 and external system 220.

Implantable medical device 210 includes a neurostimulation circuit and/or a sensing circuit. In one embodiment, implantable medical device 210 senses a physiological signal and transmits data to implantable medical device 110 via telemetry link 212 to allow implantable medical device 110 to use the sensed physiological signal to control the delivery of the neurostimulation. In one embodiment, implantable medical device 210 delivers neurostimulation by executing a stimulation algorithm for modulating a cardiovascular function. For example, as illustrated in FIG. 2, implantable medical device 110 is implanted in a vicinity of nerve 104, and implantable medical device 210 is implanted in a vicinity of nerve 103. By controlling stimulation parameters, parasympathetic stimulation is delivered from implantable medical device 110 to increase or decrease the parasympathetic tone, and sympathetic stimulation is delivered from implantable medical device 210 to increase or decrease the sympathetic tone. In one embodiment, implantable medical devices 110 and 210 are coordinated using telemetry link 212 to control autonomic balance in body 102 by delivering one or more of parasympathetic excitation, parasympathetic inhibition, sympathetic excitation, and sympathetic inhibition.

Telemetry link 212 provides intra-body telemetry between implantable medical devices. In one embodiment, telemetry link 212 is a far-field radiofrequency (RF) telemetry link. In another embodiment, telemetry link 212 is an ultrasonic telemetry link.

External system 220 communicates with implantable medical device 110 via telemetry link 214, thus providing for access to implantable medical devices 110 and 210 by a physician or other caregiver. In the illustrated embodiment, implantable medical device 110 functions as a central implantable device in system 200 that coordinates the operation of implantable medical devices 210 via telemetry link 212. In one embodiment, external system 220 includes a programmer. In another embodiment, external system 220 is a patient management system including an external device communicating with implantable medical device 110 via telemetry link 214, a remote device in a relatively distant location, and a telecommunication network linking the external device and the remote device. The patient management system allows access to implantable medical devices 110 and 210 from a remote location, for purposes such as monitoring patient status and adjusting therapies. In one embodiment, telemetry link 214 is an inductive telemetry link. In another embodiment, telemetry link 214 is a far-field RF telemetry link. In another embodiment, telemetry link 214 is an ultrasonic telemetry link (with an external acoustic coupler attached to the surface of body 102 during a telemetry session to use tissue of body 102 as the media for acoustic signal transmission). Telemetry link 214 provides for data transmission from implantable medical device 110 to external system 220. This includes, for example, transmitting real-time physiological data acquired by implantable medical devices 110 and/or 210, extracting physiological data acquired by and stored in implantable medical devices 110 and/or 210, extracting patient history data such as occurrences of arrhythmias and therapy deliveries recorded in implantable medical devices 110 and/or 210, and/or extracting data indicating an operational status of implantable medical devices 110 and/or 210 (e.g., battery status). Telemetry link 214 also provides for data transmission from external system 220 to implantable medical devices 110 and/or 210. This includes, for example, programming implantable medical devices 110 and/or 210 to acquire physiological data, programming implantable medical devices 110 and/or 210 to perform at least one self-diagnostic test (such as for a device operational status), and/or programming implantable medical devices 110 and/or 210 to deliver neurostimulation and/or to adjust the delivery of the neurostimulation.

In various embodiments, system 200 includes a network of miniature implantable medical devices such as implantable medical devices 110 and 210 to modulate one or more cardiovascular functions using neurostimulation. The miniature implantable medical devices each deliver neurostimulation and/or sense a physiologic signal.

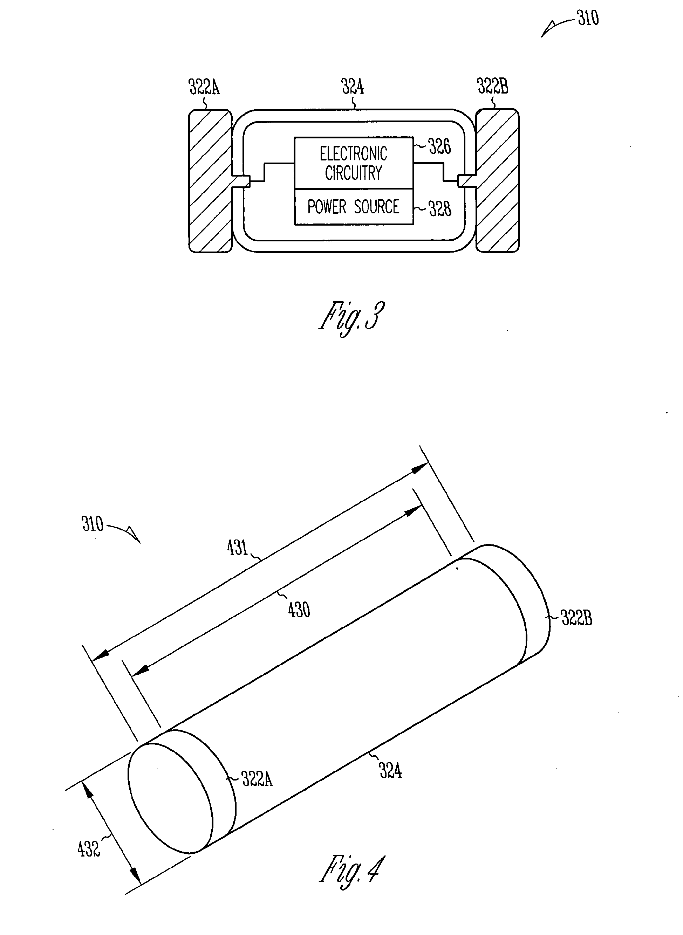

FIG. 3 is an illustration of an embodiment of a miniature implantable medical device 310, which represents a specific embodiment of implantable medical device 110 or implantable medical device 210. Implantable medical device 310 includes electronic circuitry 326, a power source 328, an implantable capsule 324, and electrodes 322A-B. Electronic circuitry 326 delivers neurostimulation and/or senses a physiologic signal. Power source 328 supplies energy to electronic circuitry 326 for its operation. Implantable capsule 324 includes a wall forming a chamber to house electronic circuitry 326 and power source 328. In the illustrated embodiment, electrodes 322A-B are each electrically coupled to electronic circuitry 326 and passing through the wall of implantable capsule 324 at one of the opposite ends of implantable capsule 324. In another embodiment, one or both of electrodes 322A-B are each electrically coupled to electronic circuitry 326 through a lead. In various embodiments, electrodes 322A-B function as a pair of stimulation electrodes for delivering neurostimulation and/or a pair of sensing electrodes for sensing a physiologic signal.

FIG. 4 is another illustration of implantable medical device 310 showing its dimensions. In the illustrated embodiment, implantable medical device 310 has an approximately cylindrical elongate shape formed by implantable capsule 324 coupled between electrodes 322A-B. Implantable capsule 324 has an approximately cylindrical elongate body. In one embodiment, implantable medical device 310 has a length 431 between approximately 5 mm and 30 mm. Implantable capsule 324 (implantable medical device 310 without electrodes 322A-B) has a length 430 between approximately 5 mm and 25 mm. Implantable medical device 310 (as well as implantable capsule 324) has a diameter 432 between approximately 2 mm and 10 mm.

In one embodiment, implantable medical device 310 is configured to be injected through a hollow injection device having an end configured to reach a stimulation target region in body 102. Examples of the hollow injection device include a hollow needle and a hollow catheter.

In one embodiment, implantable medical device 310 includes a BION® microstimulator (Advanced Bionics Corporation, a company of Boston Scientific Corporation, Sylmar, California, U.S.A.). The BION® microstimulator is discussed in, for example, U.S. Patent Nos. 5,193,539; 5193,540; 5,312,439; 5,324,316; 5,405,367; 6,051,017; and 6,185,452, which are incorporated by reference herein in their entireties.

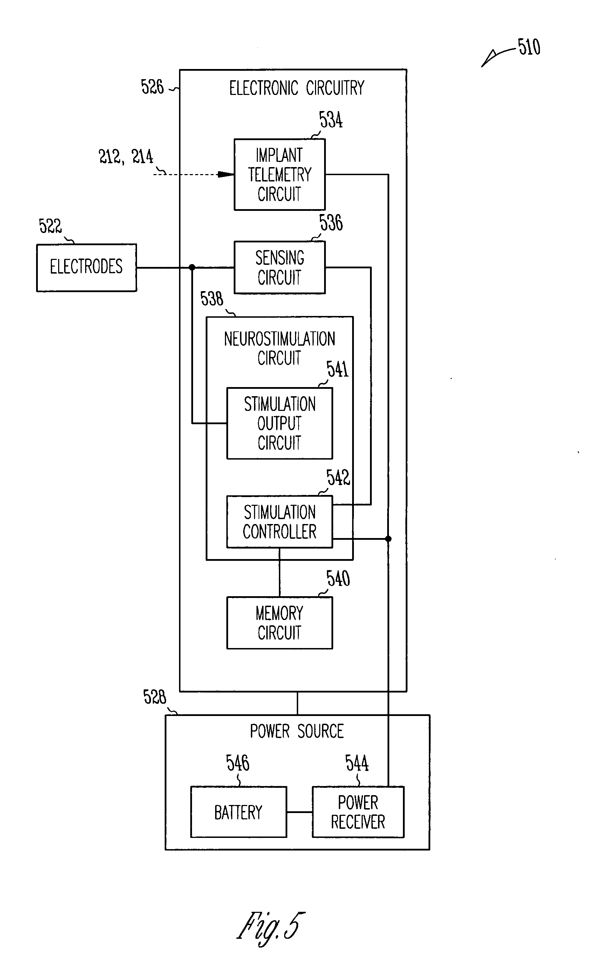

FIG. 5 is a block diagram illustrating an embodiment of a circuit 510, which represents an example of a circuit of implantable medical device 310. Circuit 510 includes electrodes 522, electronic circuitry 526, and power source 528.

Electrodes 522 include at least a pair of electrodes allowing for delivery of neurostimulation and/or physiologic signal sensing, such as electrodes 322A-B. In one embodiment, electrodes 522 include more than two electrodes from which a pair of electrodes is selected at a time to deliver the neurostimulation, for example, to allow selective neural activation and/or optimization of neurostimulation parameters.

Electronic circuitry 526 is an embodiment of electronic circuitry 326. In the illustrated embodiment, electronic circuitry 526 includes an implant telemetry circuit 534, a sensing circuit 536, a neurostimulation circuit 538, and a memory circuit 540. In various embodiments, electronic circuitry 526 is capable of performing one or both of sensing and neurostimulation delivery functions. In another embodiment, circuit 510 is a circuit of a miniature implantable sensing device and includes at least implant telemetry circuit 534, and sensing circuit 536. In another embodiment, circuit 510 is the circuit of a miniature implantable neurostimulator and includes implant telemetry circuit 534, neurostimulation circuit 538, and memory circuit 540.

Implant telemetry circuit 534 allows circuit 510 to communicate with external system 220 via telemetry link 214 and/or implantable medical device 210 via telemetry link 212. Sensing circuit 536 senses a physiologic signal such as a neural signal or a cardiac signal through electrodes 522. Neurostimulation circuit 538 includes a stimulation output circuit 541 and a stimulation controller 542. Stimulation output circuit 541 delivers the neurostimulation to body 102 through electrodes 522. In one embodiment, stimulation output circuit 541 includes a pulse output circuit that delivers electrical stimulation pulses. In other embodiments, stimulation output circuit 541 includes a light emitter to deliver light stimulation, an ultrasonic transducer to deliver ultrasonic stimulation, or a magnetic field generator to deliver magnetic stimulation. Stimulation controller 542 controls the delivery of the neurostimulation by executing a stimulation algorithm for modulating a cardiovascular function. For example, the stimulation algorithm is defined by a plurality of stimulation parameters selected to provide one or more of a cardiac remodeling control therapy, an anti-arrhythmia therapy, and an anti-hypertension therapy. In one embodiment, the stimulation algorithm includes stimulation parameters for control of delivery of the electrical stimulation pulses. Examples of the stimulation parameters for controlling the delivery of electrical stimulation pulses include pulse amplitude, pulse width, stimulation frequency (or inter-pulse interval), periodic dose, and duty cycle. The pulse amplitude and pulse width are selected to ensure that each pulse elicits an action potential in the target nerve. In one embodiment, the stimulation frequency is between approximately 0.1 and 200 Hz, with between approximately 1 and 30 Hz as a specific example for modulating cardiovascular functions. The periodic dose is a time interval during which a patient is treated with neurostimulation for each predetermined period. In one embodiment, the predetermined period is a day, and the periodic dose is a daily dose. The duty cycle is the duty cycle of the neurostimulation during the time interval during of the period dose. For example, if the patient is to receive a neurostimulation therapy for two hours each day, the periodic dose is 2 hours/day (or the daily dose is 2 hours). If the neurostimulation during those two hours is delivered intermittently with alternating on- and off-periods, the duty cycle is the ratio of the on-period to the sum of the on-period and the off-period. In one embodiment, the daily dose is between approximately 0.5 and 24 hours. In one embodiment, the duty cycle is between approximately 10 and 50%. The on-period is between approximately 10 and 120 seconds, and the off-period is between approximately 50 and 120 seconds. Memory circuit 540 stores the stimulation algorithm including the stimulation parameters. In one embodiment, memory circuit 540 also stores the history of delivery of the neurostimulation.

Power source 528 is a specific embodiment of power source 328. In the illustrated embodiment, power source 528 includes a power receiver 544 and a battery 546. In one embodiment, battery 546 is a rechargeable battery. Power receiver 544 receives inductively transmitted or acoustically transmitted power via telemetry link 214 and converts the received power to direct-current (DC) power to supply electronic circuitry 526 and/or recharge rechargeable battery 546. In another embodiment, power source 528 includes power receiver 544 but not a battery, and the power is transmitted via telemetry link 214 during delivery of the neurostimulation therapy. Such a device may be suitable, for example, when the periodic dose is low. In another embodiment, power source 528 includes a non-rechargeable battery 546, for example, when a short-term neurostimulation therapy is intended.

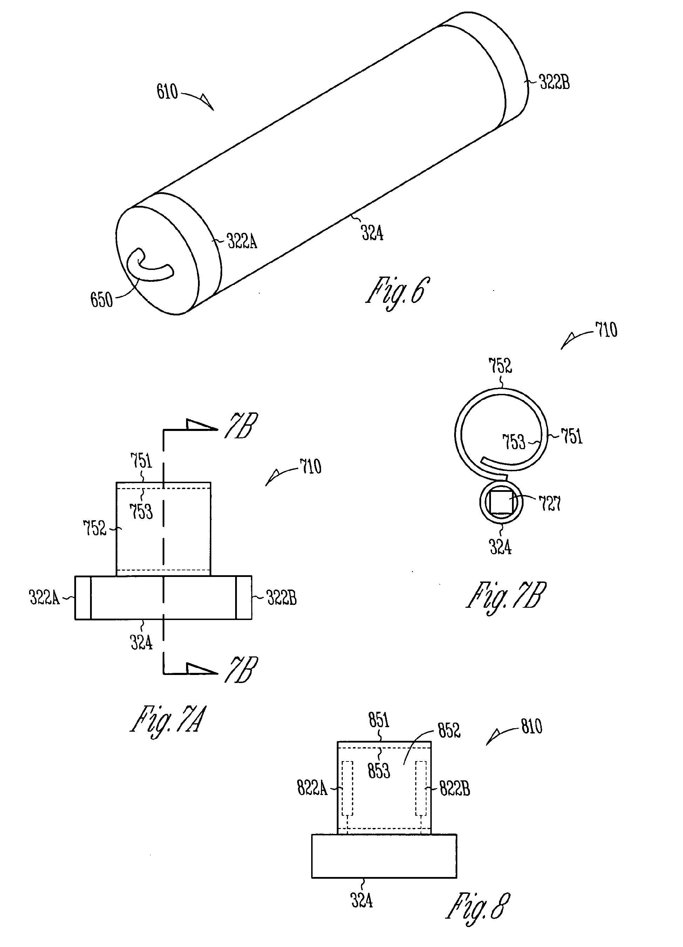

FIGS. 6-15 illustrate various embodiments of a fixation device that prevents a miniature implantable medical device from unintended displacement after implantation into body 102. The fixation device stabilizes the relative position between the miniature implantable medical device and the stimulation target in body 102. In FIGS. 6-15, the miniature implantable medical devices (610, 710, 810, 910, 1010, 1110, 1210, 1310, and 1410) each represent an embodiment of the fixation device coupled to an embodiment of implantable medical device 310. That is, as illustrated in FIGS 6-15, implantable capsule 324 houses electronic circuitry such as electronic circuitry 510 as discussed above.

FIG. 6 is an illustration of an embodiment of an implantable medical device 610, which includes implantable capsule 324 coupled between electrodes 322A-B and a suture loop 650. Suture loop 650 allows for fixation of implantable medical device 610 to a position in body 102 by suturing to tissue of body 102. In the illustrated embodiment, suture loop 650 is formed on electrode 322A. In various embodiments, implantable medical device 610 includes one or more suturing loops formed on each or both of electrodes 322A-B and/or the wall of implantable capsule 324.

FIGS. 7-10 illustrate embodiments of a fixation device that includes one or more cuffs configured to wrap around an elongate structure in body 102. In one embodiment, the elongate structure is an approximately cylindrical structure, such as a nerve or blood vessel. In other embodiments, the elongate structure includes any structure suitable for being grasped by the one or more cuffs. In one embodiment, the cuffs (752, 852, 956A-B, and 1056A-B) discussed below with reference to FIGS. 7-10 are each configured to accommodate a range of circumference of the elongate structure between approximately 5 mm and 100 mm. The circumference of the elongate structure is the circumference of a cross-sectional plane of the elongate structure where the cuff is applied.

FIG. 7A is a side view illustration, and FIG. 7B is a cross-sectional view illustration, of an embodiment of an implantable medical device 710, which includes implantable capsule 324 coupled between electrodes 322A-B and a fixation cuff 752. Implantable capsule 324 houses a device 727 that includes the electronic circuitry and power source of implantable medical device 710, such as electronic circuitry 326 and power source 328. Cuff 752 includes a wall having an exterior surface 751 and an interior surface 753 and is configured to wrap around the elongate structure in body 102. External surface 751 is affixed onto the wall of implantable capsule 324. Interior surface 753 is in contact with the elongate structure after implantation of implantable medical device 710.

FIG. 8 is an illustration of an embodiment of an implantable medical device 810, which includes implantable capsule 324 coupled to electrodes 822A-B and a fixation cuff 852. Electrodes 822A-B have the same functions as electrodes 322A-B but are incorporated onto cuff 852. Cuff 852 includes a wall having an exterior surface 851 and an interior surface 853 and is configured to wrap around the elongate structure in body 102. External surface 851 is affixed onto the wall of implantable capsule 324. Interior surface 853 is in contact with the elongate structure after implantation of implantable medical device 810. Electrodes 822A-B are incorporated onto interior surface 853. In other words, implantable medical device 810 is similar to implantable medical device 710 except that electrodes are incorporated onto the fixation cuff.

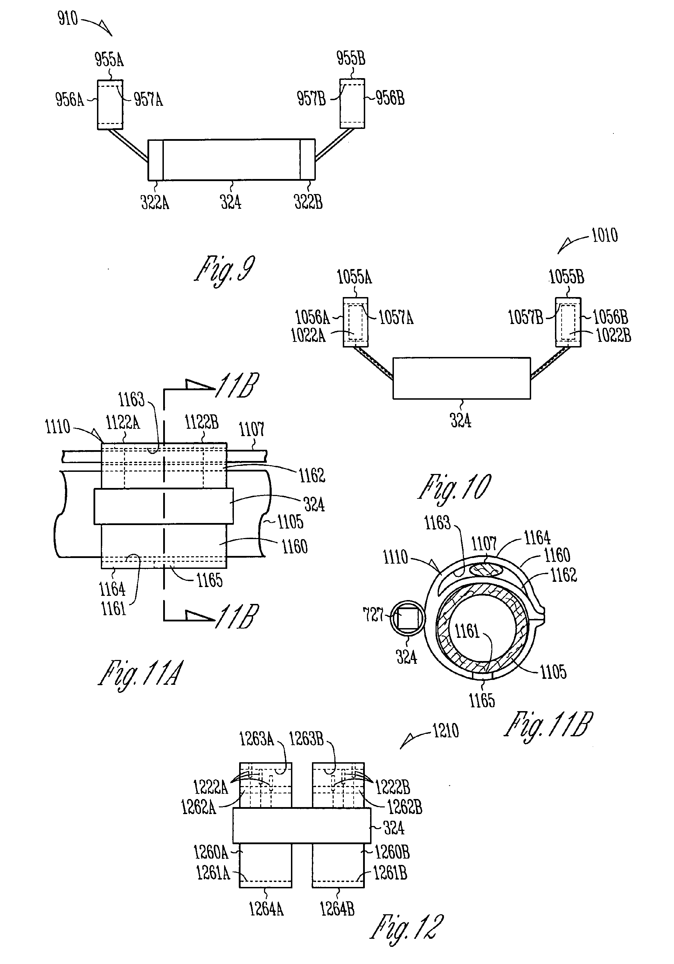

FIG. 9 is an illustration of an embodiment of an implantable medical device 910, which includes implantable capsule 324 coupled between electrodes 322A-B and coupled to fixation cuffs 956A-B. In the illustrated embodiment, cuffs 956A-B are each connected to one of electrodes 322A-B through a rigid or flexible lead. In another embodiment, cuffs 956A-B are each connected to implantable capsule 324 through a rigid or flexible lead. In the illustrated embodiment, cuff 956A includes a wall having an exterior surface 955A and an interior surface 957A, and cuff 956B includes a wall having an exterior surface 955B and an interior surface 957B. Cuffs 956A-B are each configured to wrap around the elongate structure in body 102. Interior surface 957A-B are each in contact with the elongate structure after implantation of implantable medical device 910.

FIG. 10 is an illustration of an embodiment of an implantable medical device 1010, which includes implantable capsule 324 coupled to electrodes 1022A-B and fixation cuffs 1056A-B. Electrodes 1022A-B have the same functions as electrodes 322A-B but are each incorporated onto one of cuffs 1056A-B. Cuffs 1056A-B are each connected to implantable capsule 324 through a rigid or flexible lead. Cuff 1056A includes a wall having an exterior surface 1055A and an interior surface 1057A, and cuff 1056B includes a wall having an exterior surface 1055B and an interior surface 1057B. Cuffs 1056A-B are each configured to wrap around the elongate structure in body 102. Interior surface 1057A-B are each in contact with the elongate structure after implantation of implantable medical device 1010. In other words, implantable medical device 1010 is similar to implantable medical device 910 except that the electrodes are each incorporated onto a fixation cuff.

FIGS. 11 and 12 illustrate embodiments of a vascular fixation device that includes one or more cuffs configured to wrap around a blood vessel for delivering the neurostimulation to a target nerve adjacent to the blood vessel. In one embodiment, the cuffs (1160, and 1260A-B) discussed below with reference to FIGS. 11 and 12 are each configured to accommodate a range of circumference of the blood vessel between approximately 5 mm and 100 mm. The circumference of the blood vessel is the circumference of a cross-sectional plane of the blood vessel where the cuff is applied.

FIG. 11A is a side view illustration, and FIG. 11B is a cross-sectional view illustration, of an embodiment of an implantable medical device 1110, which includes implantable capsule 324 coupled to electrodes 1122A-B and a vascular fixation cuff 1160. Cuff 1160 allows for fixation of implantable medical device 1110 to a blood vessel 1105 to deliver the neurostimulation to a nerve 1107, which is adjacent to blood vessel 1105. Cuff 1160 is configured to wrap around blood vessel 1105 such that electrodes 1122A-B are positioned stably to deliver the neurostimulation to nerve 1107. In the illustrated embodiment, cuff 1160 includes a spacer 1162 separating nerve 1107 from blood vessel 1105, an exterior surface 1164, a first interior surface 1161, and a second interior surface 1163. In various embodiments, spacer 1162 may be optional, depending on, for example, the anatomy of the implantation site of implantable medical device 1110. After implantation of implantable medical device 1110, interior surface 1161 is in contact with blood vessel 1105, and interior surface 1163 is in contact with nerve 1107. Examples of implantable medical device 1110 are discussed in U.S. Patent Application Serial No. 11/151,103, entitled "VASCULARLY STABILIZED PERIPHERAL NERVE CUFF ASSEMBLY", filed June 13, 2005, assigned to Cardiac Pacemakers Incorporated, which is incorporated by reference herein in its entirety.

In the illustrated embodiment, implantable medical device 1110 includes a hemodynamic sensor 1165 incorporated onto cuff 1160. Hemodynamic sensor 1165 senses one or more hemodynamic signals, such as blood pressure, blood flow, blood gas levels, and heart rate.

FIG. 12 is an illustration of an embodiment of an implantable medical device 1210, which includes implantable capsule 324 coupled to electrodes 1222A-B and vascular fixation cuffs 1260A-B. Cuff 1260A-B allow for fixation of implantable medical device 1210 to a blood vessel 1105 to deliver the neurostimulation to a nerve 1107 adjacent to blood vessel 1105. Cuffs 1260A-B are configured to wrap around blood vessel 1105 such that electrodes 1222A-B are positioned stably to deliver the neurostimulation to nerve 1107. Electrodes 1222A-B each include an electrode array to allow for selective neural activation by selecting an electrode from electrode 1222A and another electrode from electrode 1222B, when nerve 1107 is a nerve bundle including multiple nerves. In the illustrated embodiment, cuff 1260A includes a spacer 1262A separating nerve 1107 from blood vessel 1105, an exterior surface 1264A, a first interior surface 1261 A, and a second interior surface 1263A. Cuff 1260B includes a spacer 1262B separating nerve 1107 from blood vessel 1105, an exterior surface 1264B, a first interior surface 1261B, and a second interior surface 1263B. In various embodiments, spacers 1162A-B may be optional, depending on, for example, the anatomy of the implantation site of implantable medical device 1210. After implantation of implantable medical device 1210, interior surfaces 1261A-B are in contact with blood vessel 1105, and interior surfaces 1263A-B are in contact with nerve 1107.

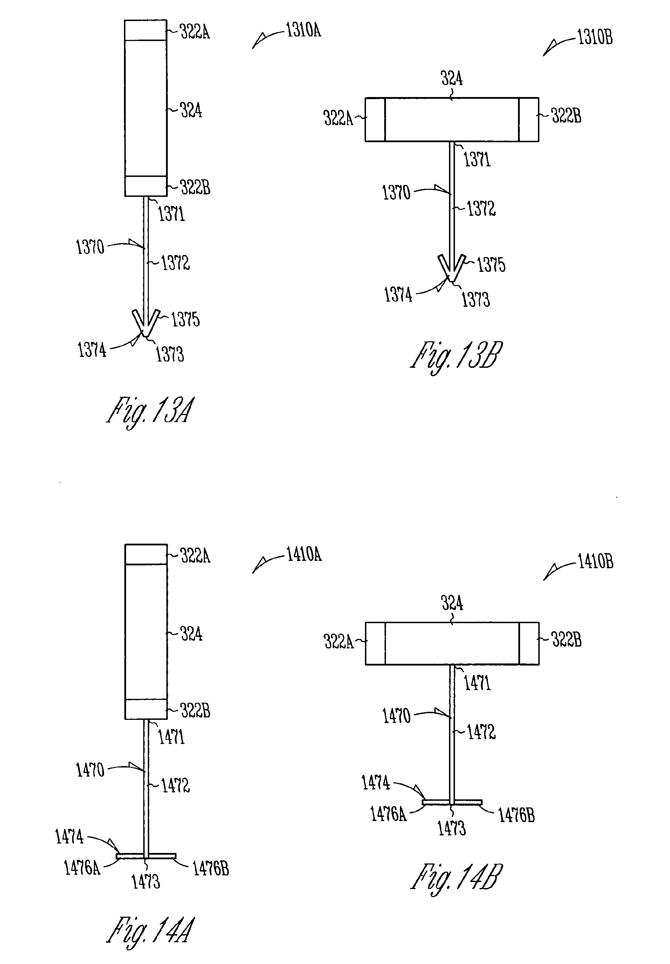

FIGS. 13-15 illustrate embodiments of a transmural fixation device that includes an anchoring device configured to transmurally fix a miniature implantable medical device to an internal structure of body 102 that has a cavity. The internal structure has a wall including an exterior surface and an interior surface, and the cavity is defined by the interior surface. Examples of such an internal structure include stomach, urinary bladder, heart, and any tubular structure including a lumen being the cavity. Examples of such a tubular structure include blood vessels, esophagus, intestines, and lymphatic vessels.

FIG. 13A is an illustration of an embodiment of an implantable medical device 1310A, which includes implantable capsule 324 coupled to a transmural fixation device 1370. Transmural fixation device 1370 is configured to transmurally fix implantable medical device 1310A to the internal structure of body 102 that has a cavity. Transmural fixation device 1370 includes a proximal end 1371, a distal end 1373, and an elongate transmural body 1372 between proximal 1371 end and distal end 1373. In the illustrated embodiment, proximal end 1371 is coupled to electrode 322B. In other embodiments, proximal end 1371 may be coupled to electrode 322A or implantable capsule 324. Distal end 1373 includes an anchoring device 1374 with barbs 1375. Anchoring device 1374 is configured to pierce the wall of the internal structure from its exterior surface to enter the cavity of the internal structure and deployed in the cavity against the interior surface of the wall. In one embodiment, elongate transmural body 1372 includes a rigid or flexible wire having a length between approximately 5 mm and 30 mm. In another embodiment, implantable medical device 1310A includes two or more transmural fixation devices 1370 coupled to implantable capsule 324 and/or electrodes 322A-B. As illustrated in FIG. 13A, elongate transmural body 1372 is approximately parallel to the longitudinal axis of implantable capsule 324. In a specific embodiment, elongate transmural body 1372 extends from the longitudinal axis of implantable capsule 324.

FIG. 13B is an illustration of an embodiment of an implantable medical device 1310B, which also includes implantable capsule 324 coupled to transmural fixation device 1370. Implantable medical device 1310B is substantially similar to implantable medical device 1310A except that elongate transmural body 1372 is approximately perpendicular to the longitudinal axis of implantable capsule 324. In the illustrated embodiment, proximal end 1371 is coupled to implantable capsule 324. In another embodiment, proximal end 1371 is coupled to one of electrodes 322A-B. In various embodiments, an implantable medical device includes one or more transmural fixation devices 1370 each coupled to any portion of implantable capsule 324 and electrodes 322A-B, with elongate transmural body 1372 extending in any direction relative to the longitudinal axis of implantable capsule 324. The specific configuration is designed and selected for the anatomy at and near the stimulation site.

FIG. 14A is an illustration of an embodiment of an implantable medical device 1410A, which includes implantable capsule 324 coupled to a transmural fixation device 1470. Transmural fixation device 1470 is configured to transmurally fix implantable medical device 1410A to the internal structure of body 102 that has a cavity. Transmural fixation device 1470 includes a proximal end 1471, a distal end 1473, and an elongate transmural body 1472 between proximal end 1471 and distal end 1473. In the illustrated embodiment, proximal end 1471 is coupled to electrode 322B. In another embodiment, proximal end 1471 is coupled to electrode 322A or implantable capsule 324. Distal end 1473 includes an anchoring device 1474. Anchoring device 1474 includes arms 1476A-B and is configured to pierce the wall of the internal structure from its exterior surface with arms 1476A-B in a folded (restrained) state, enter the cavity of the internal structure, and deploy in the cavity against the interior surface of the wall with arms 1476A-B in an expanded (unrestrained) state. In the illustrated embodiment, anchoring device 1474 includes two arms 1476A-B each approximately perpendicular to elongate transmural body 1472 when being in their expanded (unrestrained) state. In various embodiments, anchoring device 1474 includes two or more arms such as arms 1476A-B. In one embodiment, elongate transmural body 1472 includes a rigid or flexible wire having a length between approximately 5 mm and 30 mm, and arms 1476A-B each have a length between approximately 1 and 5 mm. In another embodiment, implantable medical device 1410A includes two or more transmural fixation devices 1470 coupled to implantable capsule 324 and/or electrodes 322A-B. In a specific embodiment, elongate transmural body 1472 extends from the longitudinal axis of implantable capsule 324.

FIG. 14B is an illustration of an embodiment of an implantable medical device 1410B, which also includes implantable capsule 324 coupled to transmural fixation device 1470. Implantable medical device 1410B is substantially similar to implantable medical device 1410A except that elongate transmural body 1472 is approximately perpendicular to the longitudinal axis of implantable capsule 324. In the illustrated embodiment, proximal end 1471 is coupled to implantable capsule 324. In another embodiment, proximal end 1471 is coupled to one of electrodes 322A-B. In various embodiments, an implantable medical device includes one or more transmural fixation devices 1470 each coupled to any portion of implantable capsule 324 and electrodes 322A-B, with elongate transmural body 1472 extending in any direction relative to the longitudinal axis of implantable capsule 324. The specific configuration is designed and selected based on considerations including, for example, the location of the stimulation target and the potential invasiveness of the procedure for properly placing the implantable medical device.

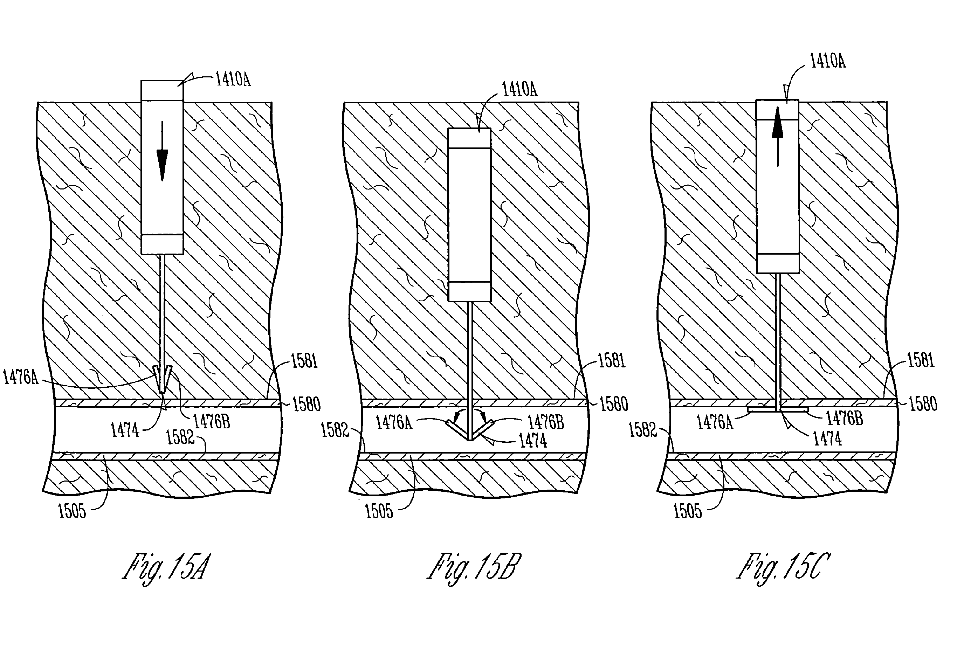

FIGS. 15A-C are illustrations of an embodiment of a method for fixing the position of implantable medical device 1410A. The illustrated method also generally applies to the fixation of implantable medical device 1410B, though a different level of invasiveness may be required. FIG. 15A-C each illustrate portions of body 102 including an internal structure 1505 having a cavity. Internal structure 1505 has a wall 1580 with an exterior surface 1581 and an interior surface 1582. Interior surface 1582 defines the cavity. In FIG. 15A, implantable medical device 1410 is advanced toward internal structure 1505 with arms 1476A-B folded (restrained) until anchoring device 1474 has pierced wall 1580 and arms 1476A-B are in the cavity defined by interior surface 1582. In FIG. 15B, arms 1476A-B expand after completely entering the cavity. In FIG. 15C, implantable medical device 1410 are pulled back to allow arms 1476A-B, now in their expanded (unrestrained) state, to deploy against interior surface 1582. Anchoring device 1474 is configured, and internal structure 1505 is selected, to ensure that the process illustrated in FIGS. 15A-C does not cause intolerable damage to internal structure 1505 or any other portion of body 102.

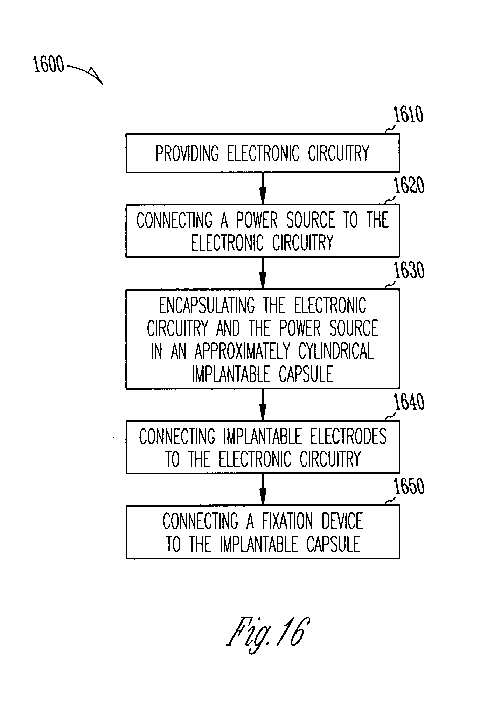

FIG. 16 is a flow chart illustrating a method 1600 for making a miniature implantable medical device. Examples of the miniature implantable medical device include implantable medical device 110, 210, 310, 510, 610, 710, 810, 910, 1010, 1110, 1210, 1310, and 1410 as discussed above.

Electronic circuitry is provided at 1610. The electronic circuitry is capable of performing one or more of sensing and therapeutic functions. In one embodiment, the electronic circuitry includes a neurostimulation circuit that delivers neurostimulation to modulate one or more cardiovascular functions. A specific example of the electronic circuitry includes electronic circuitry 526 as discussed above.

A power source is connected to the electronic circuitry at 1620. In various embodiments, the electronic circuitry is battery powered and/or powered via a wireless power transmission link. A specific example of the power source includes power source 528 as discussed above.

The electronic circuitry and the power source are encapsulated in an approximately cylindrical implantable capsule at 1630. A specific example of the implantable capsule includes implantable capsule 324 as discussed above. In one embodiment, the implantable capsule is configured to be injected into a patient's body using a hollow injection device such as a hollow needle or catheter.

Implantable electrodes are connected to the electronic circuitry at 1640. The electrodes each function as a stimulation electrode, a sensing electrode, and/or an indifferent electrode. In one embodiment, the electrodes are each affixed onto the implantable capsule or coupled to the implantable capsule through a lead. In one embodiment, the electrodes are mounted on the implantable capsule and each pass through the wall of the implantable capsule at one of the opposite ends of the implantable capsule.

A fixation device is connected to the implantable capsule at 1650. The fixation device fixes the miniature implantable medical device to a location in the body to prevent drift of the electrodes that may affect sensing and/or therapy delivery functions of the device. Specific examples of such a fixation device include suture loop 650, fixation cuffs 752, 852, 956A-B, 1056A-B, 1160, and 1260A-B, transmural fixation devices 1370 and 1470, and any combination of these devices.

It is to be understood that the above detailed description is intended to be illustrative, and not restrictive. Other embodiments will be apparent to those of skill in the art upon reading and understanding the above description. The scope of the invention should, therefore, be determined with reference to the appended claims, along with the full scope of equivalents to which such claims are entitled.

## Claims

1. An implantable medical device (1310A, 1310B, 1410A, 1410B) for use in a living body, the device comprising:
- electronic circuitry;
- an implantable capsule (324) including a wall forming a chamber housing the electronic circuitry; and
- a transmural fixation device (1370, 1470) configured to transmurally fix the implantable capsule (324) to an internal structure of the body, the internal structure having a wall and a cavity, the wall including an exterior surface and an interior surface, the cavity defined by the interior surface, the transmural fixation device including:
- a proximal end (1371, 1471) coupled to the implantable capsule;
- a distal end (1373, 1473) including an anchoring device (1374, 1474) and configured to pierce the wall from the exterior surface to enter the cavity such that the anchoring device (1374, 1474) is deployed in the cavity against the interior surface; and **characterized in that** said transmural fixation device further includes
- an elongate transmural body (1372, 1472) between the proximal end (1371, 1471) and the distal end (1373, 1473).

2. The device according to claim 1, wherein the electronic circuitry comprises a neurostimulation circuit adapted to deliver neurostimulation.

3. The device according to any of claims 1 and 2, wherein the anchoring device comprises barbs (1375).

4. The device according to any of claims 1 and 2, wherein the anchoring device comprises a plurality of arms and is configured to pierce the wall from the exterior surface with the arms in a restrained state, enter the cavity, and deploy in the cavity against the interior surface with the arms in an unrestrained state.

5. The device according to claim 4, wherein the anchoring device comprises two arms each approximately perpendicular to the elongate transmural body at the distal end of the transmural fixation device when the two arms are in the unrestrained state.

6. The device according to any of the preceding claims, wherein the implantable capsule is approximately cylindrical.

7. The device according to claim 6, wherein the implantable capsule has a length between approximately 5 mm and 25 mm and a diameter between approximately 2 mm and 10 mm.

8. The device according to any of the preceding claims, wherein the elongate transmural body comprises a wire having a length between approximately 5 mm and 30 mm.

9. The implantable medical device according to any of the preceding claims, wherein the electronic circuitry is adapted to deliver neurostimulation, the electronic circuitry including:
- a stimulation output circuit adapted to deliver the neurostimulation; and
- a stimulation controller adapted to control the delivery of the neurostimulation by executing a stimulation algorithm for modulating the cardiovascular function;
the implantable capsule including an approximately cylindrical elongate body.

10. The device according to any of the preceding claims, further comprising a power source including a rechargeable battery, and wherein the chamber is configured to house the electronic circuitry and the power source.

11. The device according to any of the preceding claims, wherein the electronic circuitry comprises a sensing circuit adapted to sense at least one physiologic signal.

12. A method for making an implantable medical device (1310A, 1310B, 1410A, 1410B), the method comprising:
- providing electronic circuitry;
- encapsulating the electronic circuitry in an implantable capsule (324);
- providing a transmural fixation device (1370, 1470) configured to transmurally fix the implantable capsule (324) to an internal structure of the body, the internal structure having a wall and a cavity, the wall including an exterior surface and an interior surface, the cavity defined by the interior surface, the transmural fixation device (1370, 1470) including a proximal end (1371, 1471), a distal end (1373, 1473), and an elongate transmural body (1372, 1472) between the proximal end (1371, 1471) and the distal end (1373, 1473), the distal end (1373, 1473) including an anchoring device (1374, 1474) and configured to pierce the wall from the exterior surface to enter the cavity such that the anchoring device (1374, 1474) is deployed in the cavity against the interior surface; and
- coupling the proximal end (1371, 1471) to the implantable capsule (324).

13. The method according to claim 12, wherein providing electronic circuitry comprises providing a neurostimulation circuit adapted to deliver neurostimulation.

14. The method according to any one of claims 12 and 13, further comprising forming the anchoring device by forming barbs at the distal end of the transmural fixation device or by forming a plurality of arms at the distal end of the transmural fixation device, the a plurality of arms configured to pierce the wall from the exterior surface with the arms in a restrained state, enter the cavity, and deploy in the cavity against the interior surface with the arms in an unrestrained state, preferably wherein forming the plurality of arms at the distal end of the transmural fixation device comprises forming two arms approximately perpendicular to the elongate transmural body at the distal end of the transmural fixation device when the two arms are in the unrestrained state.

15. The method according to any of claims 12 to 14, further comprising configuring the transmural fixation device to transmurally fix the implantable capsule to a digestive organ having the cavityor a tubular structure having a lumen being the cavityor a blood vessel.

## Patentansprüche

1. Implantierbare medizinische Vorrichtung (1310A, 1310B, 1410A, 1410B) zur Verwendung in einem lebenden Körper, welche Vorrichtung aufweist:
- eine elektronische Schaltung;
- eine implantierbare Kapsel (324), enthaltend eine Wand, die eine die elektronische Schaltung aufnehmende Kammer bildet; und
- eine die Wand durchdringende Befestigungsvorrichtung (1370, 1470), die konfiguriert ist, durch die Wand hindurchgehend die implantierbare Kapsel (324) an einer inneren Struktur des Körpers zu befestigen, wobei die innere Struktur eine Wand und einen Hohlraum hat, die Wand eine äußere Oberfläche und eine innere Oberfläche hat und der Hohlraum durch die innere Oberfläche definiert wird, welche die Wand durchdringende Befestigungsvorrichtung enthält:
- ein proximales Ende (1371, 1471), das mit der implantierbaren Kapsel gekoppelt ist;
- ein distales Ende (1373, 1473), enthaltend eine Verankerungsvorrichtung (1374, 1474) und konfiguriert, die Wand von der äußeren Oberfläche aus zu durchstechen, um in den Hohlraum einzutreten, derart, dass die Verankerungsvorrichtung (1374, 1474) sich in dem Hohlraum gegen die innere Oberfläche entfaltet; und
**dadurch gekennzeichnet, dass** die die Wand durchdringende Befestigungsvorrichtung weiterhin enthält:
- einen länglichen, durch die Wand hindurchgehenden Körper (1372, 1472) zwischen dem proximalen Ende (1371, 1471) und dem distalen Ende (1373, 1473).

2. Vorrichtung nach Anspruch 1, bei der die elektronische Schaltung eine Neurostimulationsschaltung umfasst, die ausgestaltet ist, eine Neurostimulation zu liefern.

3. Vorrichtung nach einem der Ansprüche 1 und 2, bei der die Verankerungsvorrichtung Stachel (1375) aufweist.

4. Vorrichtung nach einem der Ansprüche 1 und 2, bei der die Verankerungsvorrichtung mehrere Arme aufweist und konfiguriert ist, die Wand von der äußeren Oberfläche aus mit den Armen in einem angelegten Zustand zu durchstechen, in den Hohlraum einzutreten und sich in dem Hohlraum gegen die innere Oberfläche mit den Armen in einem ausgebreiteten Zustand zu entfalten.

5. Vorrichtung nach Anspruch 4, bei der die Verankerungsvorrichtung zwei Arme jeweils angenähert senkrecht zu dem länglichen, durch die Wand hindurchgehenden Körper an dem distalen Ende der die Wand durchdringenden Befestigungsvorrichtung, wenn die beiden Arme in dem ausgebreiteten Zustand sind, aufweist.

6. Vorrichtung nach einem der vorhergehenden Ansprüche, bei der die implantierbare Kapsel angenähert zylindrisch ist.

7. Vorrichtung nach Anspruch 6, bei der die implantierbare Kapsel eine Länge zwischen angenähert 5 mm und 25 mm und einen Durchmesser zwischen angenähert 2 mm und 10 mm hat.

8. Vorrichtung nach einem der vorhergehenden Ansprüche, bei der der längliche, durch die Wand hindurchgehende Körper einen Draht mit einer Länge zwischen angenähert 5 mm und 30 mm aufweist.

9. Implantierbare medizinische Vorrichtung nach einem der vorhergehenden Ansprüche, bei der die elektronische Schaltung ausgestaltet ist, eine Neurostimulation zu liefern, welche elektronische Schaltung enthält:
- eine Stimulationsausgabeeinheit, die ausgestaltet ist, die Neurostimulation zu liefern; und
- eine Stimulationssteuervorrichtung, die ausgestaltet ist, die Lieferung der Neurostimulation durch Durchführen eines Stimulationsalgorithmus zum Modulieren der kardiovaskulären Funktion zu steuern;
wobei die implantierbare Kapsel einen angenähert zylindrischen länglichen Körper enthält.

10. Vorrichtung nach einem der vorhergehenden Ansprüche, weiterhin eine eine wiederaufladbare Batterie enthaltende Energiequelle aufweisend, wobei die Kammer konfiguriert ist, die elektronische Schaltung und die Energiequelle aufzunehmen.

11. Vorrichtung nach einem der vorhergehenden Ansprüche, bei der die elektronische Schaltung eine Erfassungsschaltung aufweist, die ausgestaltet ist, zumindest ein physiologisches Signal zu erfassen.

12. Verfahren zum Herstellen einer implantierbaren medizinischen Vorrichtung (1310A, 1310B, 1410A, 1410B), welches Verfahren aufweist:
- Bereitstellen einer elektronischen Schaltung;
- Einkapseln der elektronischen Schaltung in einer implantierbaren Kapsel (324);
- Bereitstellen einer eine Wand durchdringenden Befestigungsvorrichtung (1370, 1470), die konfiguriert ist, durch eine Wand hindurchgehend die implantierbare Kapsel (324) an einer inneren Struktur des Körpers zu befestigen, welche innere Struktur eine Wand und einen Hohlraum hat, wobei die Wand eine äußere Oberfläche und eine innere Oberfläche hat, der Hohlraum durch die innere Oberfläche definiert wird, die durch eine Wand hindurchgehende Befestigungsvorrichtung (1370, 1470) ein proximales Ende (1371, 1471), ein distales Ende (1373, 1473) und einen länglichen, eine Wand durchdringenden Körper (1372, 1472) zwischen dem proximalen Ende (1371, 1471) und dem distalen Ende (1373, 1473) enthält, das distale Ende (1373, 1473) eine Verankerungsvorrichtung (1374, 1474) enthält und konfiguriert ist, die Wand von der äußeren Oberfläche aus zu durchstechen, um derart in den Hohlraum einzutreten, dass die Verankerungsvorrichtung (1374, 1474) sich in dem Hohlraum gegen die innere Oberfläche entfaltet; und
- Koppeln des proximalen Endes (1371, 1471) an die implantierbare Kapsel (324).

13. Verfahren nach Anspruch 12, bei dem das Bereitstellen der elektronischen Schaltung das Bereitstellen einer Neurostimulationsschaltung, die ausgestaltet ist, eine Neurostimulation zu liefern, aufweist.

14. Verfahren nach einem der Ansprüche 12 und 13, weiterhin aufweisend: Bilden der Verankerungsvorrichtung durch Bilden von Stacheln an dem distalen Ende der die Wand durchdringenden Befestigungsvorrichtung oder durch Bilden mehrerer Arme an dem distalen Ende der durch die Wand hindurchgehenden Befestigungsvorrichtung, wobei die mehreren Arme konfiguriert sind zum Durchstechen der Wand von der äußeren Oberfläche aus mit den Armen in einem angelegten Zustand, Eintreten in den Hohlraum und Entfalten in dem Hohlraum gegen die innere Oberfläche mit den Armen in einem ausgebreiteten Zustand, wobei das Bilden der mehreren Arme an dem distalen Ende der die Wand durchdringenden Befestigungsvorrichtung das Bilden von zwei Armen angenähert senkrecht zu dem länglichen, die Wand durchdringenden Körper an dem distalen Ende der durch die Wand hindurchgehenden Befestigungsvorrichtung, wenn die beiden Arme in dem ausgebreiteten Zustand sind, aufweist.

15. Verfahren nach einem der Ansprüche 12 bis 14, weiterhin aufweisend das Konfigurieren der durch die Wand hindurchgehenden Befestigungsvorrichtung, um die implantierbare Kapsel durch die Wand hindurchgehend an einem Verdauungsorgan mit dem Hohlraum oder einer rohrförmigen Struktur mit einem Lumen, das der Hohlraum oder ein Blutgefäß ist, zu befestigen.

## Revendications

1. Dispositif médical implantable (1310A, 1310B, 1410A, 1410B) pour une utilisation dans un corps vivant, le dispositif comprenant :
un circuit électronique ;
une capsule implantable (324) incluant une paroi formant une chambre logeant le circuit électronique ; et
un dispositif de fixation transmurale (1370, 1470) configuré pour fixer transmuralement la capsule implantable (324) à une structure interne du corps, la structure interne comportant une paroi et une cavité, la paroi incluant une surface extérieure et une surface intérieure, la cavité étant définie par la surface intérieure, le dispositif de fixation transmurale incluant :
une extrémité proximale (1371, 1471) couplée à la capsule implantable ;
une extrémité distale (1373, 1473) incluant un dispositif d'ancrage (1374, 1474) et configurée pour percer la paroi depuis la surface extérieure afin de pénétrer dans la cavité de telle sorte que le dispositif d'ancrage (1374, 1474) soit déployé dans la cavité contre la surface extérieure ; et
**caractérisé en ce que** ledit dispositif de fixation transmurale inclut en outre :
un corps transmural allongé (1372, 1472) entre l'extrémité proximale (1371, 1471) et l'extrémité distale (1373, 1473).

2. Dispositif selon la revendication 1, dans lequel le circuit électronique comprend un circuit de neurostimulation adapté pour délivrer une neurostimulation.

3. Dispositif selon l'une quelconque des revendications 1 et 2, dans lequel le dispositif d'ancrage comprend des barbes (1375).

4. Dispositif selon l'une quelconque des revendications 1 et 2, dans lequel le dispositif d'ancrage comprend une pluralité de bras et est configuré pour percer la paroi depuis la surface extérieure, les bras étant dans un état restreint, pour pénétrer dans la cavité et pour se déployer dans la cavité contre la surface extérieure, les bras étant dans un état non restreint.

5. Dispositif selon la revendication 4, dans lequel le dispositif d'ancrage comprend deux bras dont chacun est approximativement perpendiculaire au corps transmural allongé au niveau de l'extrémité distale du dispositif de fixation transmurale lorsque les deux bras sont dans l'état de non restreint.

6. Dispositif selon l'une quelconque des revendications précédentes, dans lequel la capsule implantable est approximativement cylindrique.

7. Dispositif selon la revendication 6, dans lequel la capsule implantable présente une longueur entre approximativement 5 mm et 25 mm et un diamètre entre approximativement 2 mm et 10 mm.

8. Dispositif selon l'une quelconque des revendications précédentes, dans lequel le corps transmural allongé comprend un fil présentant une longueur entre approximativement 5 mm et 30 mm.

9. Dispositif médical implantable selon l'une quelconque des revendications précédentes, dans lequel le circuit électronique est adapté pour délivrer la neurostimulation, le circuit électronique incluant :
un circuit de sortie de stimulation adapté pour délivrer la neurostimulation ; et
un contrôleur de stimulation adapté pour contrôler la délivrance de la neurostimulation en exécutant un algorithme de stimulation pour moduler la fonction cardiovasculaire,
la capsule implantable incluant un corps allongé approximativement cylindrique.

10. Dispositif selon l'une quelconque des revendications précédentes, comprenant en outre une source d'alimentation incluant un accumulateur rechargeable, et dans lequel la chambre est configurée pour loger le circuit électronique et la source d'alimentation.

11. Dispositif selon l'une quelconque des revendications précédentes, dans lequel le circuit électronique comprend un circuit de détection adapté pour détecter au moins un signal physiologique.

12. Procédé pour fabriquer un dispositif médical implantable (1310A, 1310B, 1410A, 1410B), le procédé comprenant :
la fourniture d'un circuit électronique ;
l'encapsulage du circuit électronique dans une capsule implantable (324) ;
la fourniture d'un dispositif de fixation transmurale (1370, 1470) configuré pour fixer transmuralement la capsule implantable (324) à une structure interne du corps, la structure interne comportant une paroi et une cavité, la paroi incluant une surface extérieure et une surface intérieure, la cavité étant définie par la surface intérieure, le dispositif de fixation transmurale (1370, 1470) incluant une extrémité proximale (1371, 1471), une extrémité distale (1373, 1473) et un corps transmural allongé (1372, 1472) entre l'extrémité proximale (1371, 1471) et l'extrémité distale (1373, 1473), l'extrémité distale (1373, 1473) incluant un dispositif d'ancrage (1374, 1474) et étant configurée pour percer la paroi depuis la surface extérieure afin de pénétrer dans la cavité de telle sorte que le dispositif d'ancrage (1374, 1474) soit déployé dans la cavité contre la surface extérieure ; et
le couplage de l'extrémité proximale (1371, 1471) à la capsule implantable (324).

13. Procédé selon la revendication 12, dans lequel la fourniture du circuit électronique comprend la fourniture d'un circuit de neurostimulation adapté pour délivrer une neurostimulation.

14. Procédé selon l'une quelconque des revendications 12 et 13, comprenant en outre la formation du dispositif d'ancrage en formant des barbes au niveau de l'extrémité distale du dispositif de fixation transmurale ou en formant une pluralité de bras au niveau de l'extrémité distale du dispositif de fixation transmurale, les bras de la pluralité de bras étant configurés pour percer la paroi depuis la surface extérieure, les bras étant dans un état restreint, pour pénétrer dans la cavité et pour se déployer dans la cavité contre la surface extérieure, les bras étant dans un état non restreint, de façon préférable dans lequel la formation de la pluralité de bras au niveau de l'extrémité distale du dispositif de fixation transmurale comprend la formation de deux bras approximativement perpendiculaires au corps transmural allongé au niveau de l'extrémité distale du dispositif de fixation transmurale lorsque les deux bras sont dans l'état non restreint.

15. Procédé selon l'une quelconque des revendications 12 à 14, comprenant en outre la configuration du dispositif de fixation transmurale afin de fixer transmuralement la capsule implantable sur un organe de digestion comportant la cavité ou une structure tubulaire comportant une lumière qui est la cavité ou un vaisseau sanguin.
